# FASCICULE DE BREVET EUROPEEN

(11) **EP 0 800 499 B1**
(45) Date de publication et mention de la délivrance du brevet: **14.11.2001**
(21) Numéro de dépôt: 95943281.6
(22) Date de dépôt: 27.12.1995
(51) Int. Cl.: C07C 15/08, C07C 7/14

(54) **PROCEDE DE SEPARATION DE PARAXYLENE COMPORTANT AU MOINS DEUX ETAGES DE CRISTALLISATION A HAUTE TEMPERATURE**
VERFAHREN ZUR ABTRENNUNG VON PARA-XYLOL DAS WENIGSTENS ZWEI STUFEN ZUR KRISTALISIERUNG AUF HOHER TEMPERATUR
PARAXYLENE SEPARATION PROCESS COMPRISING AT LEAST TWO HIGH TEMPERATURE CRYSTALLIZATION STAGES

(30) Priorité: 29.12.1994 FR 9415897
(43) Date de publication de la demande: 15.10.1997
(73) Titulaire: INSTITUT FRANCAIS DU PETROLE, 92506 Rueil-Malmaison Cédex (FR)
(72) Inventeur: MIKITENKO, Paul, F-78590 Noisy-le-Roy (FR); MacPHERSON, Stuart, R., Piedmont, CA 94611 (US)
(74) Mandataire: Andreeff, François
(86) Numéro de dépôt international: FR9501739
(87) Numéro de publication internationale: WO9620908

(56) Documents cités:
- US-A- 5 329 061

## Description

L'invention concerne un procédé de séparation et de préparation de paraxylène à partir d'un mélange d'hydrocarbures aromatiques comportant des isomères du xylène.

Les isomères du xylène sont l'orthoxylène, le métaxylène, le paraxylène et l'éthylbenzène. L'application principale de l'invention est l'obtention de paraxylène à un degré de pureté suffisant pour la synthèse par exemple de l'acide téréphtalique, utilisé dans la préparation de fibres synthétiques et notamment du polyester.

La demanderesse a décrit dans un brevet FR 2 681 066 (US 5 284 992) un procédé de séparation et de récupération de paraxylène contenu dans une charge d'hydrocarbures comprenant essentiellement des hydrocarbures aromatiques à 8 atomes de carbone.

Ce procédé comprend la combinaison d'une étape d'enrichissement qui est une adsorption sélective sur un adsorbant en lit mobile simulé d'une charge contenant essentiellement les isomères du xylène qui permet d'enrichir substantiellement en paraxylène un premier effluent d'adsorption, et d'une purification qui est une cristallisation dans au moins une unité de cristallisation fonctionnant à haute température de l'effluent enrichi en paraxylène de façon à obtenir du paraxylène à très haute pureté, la liqueur mère étant recyclée à l'étape d'adsorption.

Cette cristallisation à haute température correspond à la seconde étape de cristallisation des procédés conventionnels de cristallisation (Chevron, Arco) qui comprennent en général une première cristallisation à basse température (-40 à-70°C) et une seconde cristallisation pour purifier à haute température (0 à -20°C par exemple) des cristaux obtenus préalablement refondus.

Par ailleurs, une seconde fraction appauvrie en paraxylène et donc enrichie en ortho et en méta-xylène ainsi qu'en éthylbenzène, délivrée par l'unité d'adsorption sélective, est envoyée dans une unité d'isomérisation pour augmenter sa concentration en paraxylène à une valeur proche de l'équilibre et sensiblement voisine ou supérieure à la composition de la charge initiale d'hydrocarbures et l'isomérat obtenu peut alors être recyclé à l'adsorption.

Dans l'enchaînement adsorption, cristallisation et isomérisation décrit, divers types d'impuretés peuvent apparaître dans les différents effluents et entraîner des perturbations dans la marche des unités qui nuisent au rendement obtenu et à la pureté du paraxylène récupéré.

Tout d'abord, lors de l'isomérisation de la fraction appauvrie en paraxylène, des hydrocarbures oléfiniques peuvent être produits en quantité variable suivant les valeurs des pressions partielles d'hydrogène introduit. La formation subséquente de polymères et leur passage dans l'unité d'adsorption peuvent entraîner de sérieux problèmes de circulation à travers l'adsorbant, voire sa désactivation.

De plus, des hydrocarbures paraffiniques et naphténiques à 8 et 9 atomes de carbone dont la volatilité est comprise entre celle du toluène, solvant de désorption par exemple, et celle des xylènes, sont des produits intermédiaires de la conversion de l'éthylbenzène en xylènes lors de l'isomérisation et leur accumulation peut s'avérer préjudiciable.

Par ailleurs, des hydrocarbures aromatiques à 9 atomes de carbone, présents en faible proportion et mal séparés dans des colonnes de distillation peuvent être nuisibles au procédé, tout comme des aldéhydes et des cétones plus lourds que la charge initiale, qui se forment lorsque de l'oxygène se trouve accidentellement dissout.

Enfin, un autre problème est lié à la présence de méthanol. Cet alcool est parfois rajouté en faible proportion dans les mélanges de xylènes à cristalliser pour éviter la cocristallisation d'eau et de paraxylène.

En effet, les mélanges de C₈ aromatiques secs sont relativement hygroscopiques et lors du passage dans les centrifugeuses de la suspension de cristaux de paraxylène dans la liqueur mère, de l'eau contenue dans l'air ambiant peut être absorbée dans la liqueur mère et cette eau peut éventuellement cristalliser en liaison avec la température de cette liqueur mère.

De plus, certains échangeurs peuvent présenter des fuites et de l'eau peut passer accidentellement dans le mélange à cristalliser.

Un objet de l'invention est d'améliorer la récupération du paraxylène produit et de minimiser le coût énergétique, notamment de l'étape de purification.

Un autre objet de l'invention est de remédier à ces inconvénients et de limiter notamment la teneur de ces diverses impuretés dans la section adsorption pour chercher à l'optimiser, dans la mesure où l'adsorbant est très sensible aux impuretés de la charge de la zone d'adsorption.

La présente invention concerne un procédé de production de paraxylène de très haute pureté à partir d'une charge contenant un mélange d'hydrocarbures aromatiques de 7 à 9 atomes de carbone dans lequel on fait circuler une partie au moins de la charge dans une zone dite d'enrichissement adaptée à enrichir une première fraction à plus de 50 % en poids de paraxylène et on purifie au moins une partie de ladite première fraction dans une zone dite de purification par au moins deux cristallisations à haute température comprise entre +10°C et -30°C dans au moins deux zones de cristallisation, le procédé étant caractérisé en ce que l'on cristallise ladite première fraction enrichie en paraxylène dans une première zone de cristallisation (70) à une température T1, on sépare les premiers cristaux d'une première liqueur mère, on récupère des premiers cristaux de paraxylène, on cristallise une partie au moins de la première liqueur mère dans une seconde zone de cristallisation (50), à une température T2 inférieure à la température T1, on sépare des seconds cristaux d'une seconde liqueur mère, on récupère des seconds cristaux de paraxylène et on mélange les premiers et les seconds cristaux de paraxylène.

Chaque étape de cristallisation peut comprendre un ou plusieurs cristalliseurs.

Par cristallisation à haute température du paraxylène, on entend une cristallisation d'une solution ou suspension de paraxylène déjà enrichie en paraxylène qui correspond à ce que la littérature appelle une étape de purification. Par exemple, le brevet US 2 866 833 mentionne une étape de purification du paraxylène à -34°C.

Selon une première variante, la zone d'enrichissement de la première fraction à au moins 30% poids en paraxylène peut être au moins une zone de cristallisation à très basse température, par exemple inférieur à -40°C, dite section de récupération (section recovery) dans laquelle on introduit une charge contenant un mélange d'hydrocarbures aromatiques à 8 atomes de carbone, comme celle décrite selon le brevet US 2 866 833 ou comme celles décrites selon le brevet US 5 329 061. Cette zone d'enrichissement délivre une suspension de cristaux qui est séparée dans une zone de séparation et les cristaux récupérés sont fondus et constituent une partie au moins de ladite première fraction à purifier par la suite. De plus, une liqueur mère résultant de la séparation peut être isomérisée dans une zone d'isomérisation et l'isomérat au moins en partie recyclé vers la zone d'enrichissement (section de récupération).

Selon une deuxième variante, la zone d'enrichissement en paraxylène peut être une zone d'adsorption sélective de la charge contenant un mélange d'hydrocarbures aromatiques à 8 atomes de carbone et délivrant la fraction enrichie en paraxylène.

Selon une troisième variante, la zone d'enrichissement en paraxylène peut être une zone de dismutation d'une charge consistant essentiellement en toluène et utilisant du catalyseur sélectivé par du coke ou du silicium selon les brevets US4.117.026, 4.097.543, 4.851.604, 5.173.461, 5.243.117 et 5.321.183. L'effluent de dismutation comprenant des xylènes, du benzène et du toluène qui n'a pas réagi, est avantageusement débarrassé par distillation du benzène et du toluène.

Il est avantageux de disposer en sortie de la zone d'enrichissement, c'est-à-dire par exemple de la zone d'adsorption sélective telle que décrite dans le brevet US 5 284 992 de la Demanderesse, d'un effluent contenant plus de 50% en poids de paraxylène et de préférence de 75 à 98%.

Il est possible de recycler la liqueur mère de cristallisation en des endroits différents de l'installation selon l'importance des teneurs en composés indésirables mais il peut être avantageux de combiner entre eux ces différents recyclages, par exemple, lorsqu'il s'agit de réutiliser des équipements existants pour la distillation de l'isomérat, un traitement à la terre ou la distillation dite de rerun et lorsque l'un de ces équipements est déjà opéré à son débit maximum.

L'étape de cristallisation de la fraction enrichie en paraxylène provenant de la zone d'enrichissement est en général réalisée, comme il a été mentionné ci-dessus, à haute température par exemple entre + 10 et -30°C et de préférence + 10 et -25°C. La température est habituellement choisie en fonction de la concentration en paraxylène que l'on souhaite avoir dans la liqueur mère, et du coût économique de l'opération de cristallisation.

Il est avantageux, notamment pour des raisons économiques de réaliser une cristallisation en plusieurs étages, par exemple une première cristallisation à une température T1 comprise entre +10 et -10°C, et une seconde cristallisation à une température T2, inférieure à T1, comprise entre 0 et -25°C.

Selon un mode de réalisation de l'invention, mettant en oeuvre une cristallisation à deux étages, on effectue une première cristallisation du paraxylène de l'étape (d) dans une première zone de cristallisation à une température T1, on récupère une suspension de premiers cristaux de paraxylène dans une première liqueur mère, on sépare les premiers cristaux de la première liqueur mère dans une première zone de séparation, on lave éventuellement les premiers cristaux avec un solvant de lavage dans la première zone de séparation, on cristallise une partie au moins de la première liqueur mère dans une seconde zone de cristallisation à une température T2 inférieure à la température T1, on récupère une suspension de seconds cristaux de paraxylène dans une seconde liqueur mère, on sépare les seconds cristaux de la seconde liqueur mère dans une seconde zone de séparation, on lave les seconds cristaux avec le solvant de lavage dans la seconde zone de séparation, on récupère une seconde liqueur mère que l'on recycle au moins en partie vers la zone d'adsorption ou de cristallisation à très basse température, on mélange les premiers et les seconds cristaux de paraxylène, on les fond complètement et on récupère un courant de paraxylène fondu.

Selon un autre mode de mise en oeuvre particulièrement avantageux, on effectue une première cristallisation du paraxylène de l'étape (d) dans une première zone de cristallisation à une température T1, on récupère une suspension de premiers cristaux de paraxylène dans une première liqueur mère, on sépare les premiers cristaux de la première liqueur mère dans une première zone de séparation, on cristallise une partie au moins de la première liqueur mère dans une seconde zone de cristallisation à une température T2 inférieure à T1, on sépare les seconds cristaux de la seconde liqueur mère dans une seconde zone de séparation, on récupère la seconde liqueur mère que l'on recycle au moins en partie vers la zone d'adsorption ou de cristallisation à très basse température, on récupère les premiers cristaux et les seconds cristaux et on les lave dans au moins une zone de séparation et de lavage à contre courant avec le solvant de lavage approprié, on récupère d'une part une liqueur de lavage qui est recyclée au moins en partie vers la première zone de cristallisation, on récupère d'autre part des cristaux de paraxylène, on les fond complètement dans une zone de fusion et on recueille un courant de paraxylène fondu.

L'invention concerne également un procédé de séparation et de récupération de paraxylène contenu dans une charge d'hydrocarbures comprenant des hydrocarbures aromatiques en C₈, le procédé comprenant les étapes suivantes :
a) on met en contact dans au moins une zone d'adsorption (8), ladite charge (1) contenant du métaxylène, du paraxylène et éventuellement de l'éthylbenzène et/ou de l'orthoxylène avec un adsorbant en présence d'un solvant de désorption approprié dans des conditions d'adsorption telles qu'on obtient une première fraction contenant du solvant, du métaxylène et éventuellement de l'éthylbenzène et/ou de l'orthoxylène et une seconde fraction contenant du solvant et essentiellement du paraxylène substantiellement enrichi ;
b) on distille (12) la première fraction pour séparer le solvant d'une part et le mélange métaxylène et éventuellement éthylbenzène, et/ou orthoxylène d'autre part ;
c) on isomérise ledit mélange dans des conditions appropriées dans une zone d'isomérisation (21) et l'on récupère un isomérat (2) que l'on recycle au moins en partie vers l'étape a) ;
d) on distille (16) la deuxième fraction et l'on récupère le solvant d'une part et du paraxylène substantiellement enrichi ;
e) on procède à une cristallisation du paraxylène de l'étape d) dans au moins une zone de cristallisation (5a, 5b) à haute température, comprise avantageusement entre + 10°C et - 25°C et l'on obtient par séparation d'une part une liqueur mère que l'on recycle (3) au moins en partie vers l'étape a) et d'autre part des cristaux de paraxylène imbibés de liqueur mère ;
f) On lave les cristaux de paraxylène avec un solvant de lavage approprié dans au moins une zone de lavage et l'on récupère des cristaux de paraxylène à un très grand degré de pureté ; le procédé étant caractérisé en ce qu'au moins une partie de la liqueur mère (3) (33) est introduite dans une colonne de distillation, on obtient une fraction contenant de la liqueur mère purifiée, fraction qui est envoyée ensuite dans la zone (8) d'adsorption.

Bien entendu on ne sortira pas du cadre de la présente invention en utilisant un autre solvant de désorption que le toluène tel notamment le paradiéthylbenzène (PDEB), ce solvant étant plus lourd que les xylènes, sera récupéré en fond de certaines colonnes alors que le toluène l'était en tête.

L'invention sera mieux comprise au vu des figures suivantes illustrant de manière non limitative plusieurs modes de mise en oeuvre de l'invention, parmi lesquelles :
**·** la figure 1 représente de manière schématique le procédé et les diverses possibilités de recyclage de l'isomérat et de la liqueur mère de cristallisation vers l'adsorption via un traitement à la terre.
· les figures 2 à 5 représentent une purification du paraxylène par cristallisation à double étage, le premier étant plus chaud que le second.

Les conditions opératoires et l'adsorption en lit mobile simulé (contre courant par exemple) sont choisies de façon que la première fraction contenant le métaxylène, l'orthoxylène et l'éthylbenzène soit un raffinat et la deuxième fraction contenant essentiellement le paraxylène soit un extrait. Ces conditions sont décrites dans le brevet US 5 284 992.

On véhicule par une ligne 1 une charge comprenant environ 20% d'éthylbenzène, 18% de paraxylène, 45% de métaxylène et 17% d'orthoxylène. On y joint par une ligne 2 un effluent recyclé dont la teneur en éthylbenzène est sensiblement plus faible, typiquement 8 à 13% et qui contient des impuretés. Par des lignes 3 et 30, on introduit un autre effluent recyclé dont la teneur en paraxylène est plus forte, typiquement 25 à 45%. Une ligne 4 récupère la charge et ces deux effluents, elle véhicule un mélange de composition approximative, paraxylène 20 à 22,5, éthylbenzène 9 à 14%, orthoxylène 20 à 22,5%, métaxylène 45 à 50% qui est introduit dans une zone d'adsorption 8 en contre-courant simulé comprenant une ou plusieurs colonnes 6 et/ou 7 remplies d'un adsorbant zéolitique, chacune des colonnes étant divisée en un nombre limité de lits, le nombre des lits de chaque colonne pouvant être compris entre 4 et 20, la productivité exprimée par rapport au paraxylène produit étant d'environ 0,07 m³ par m³ de tamis et par heure exprimée aux conditions ambiantes. On désorbe par du toluène, à raison d'environ 1,45 m³ de toluène par m³ de charge, la température opératoire étant à peu près 160°C. On soutire de cette unité par une ligne 10 un raffinat appauvri en paraxylène contenant essentiellement du toluène, du métaxylène, de l'éthylbenzène et de l'orthoxylène et par une ligne 9 un extrait de composition enrichie en paraxylène contenant essentiellement du toluène et du paraxylène, l'impureté majeure étant l'éthylbenzène. Le raffinat est introduit dans une colonne à distiller 12 (température de tête 125°C, température de fond 160°C par exemple). On soutire en tête par une ligne 14 du toluène (environ 30 % de la quantité introduite dans l'adsorption par exemple) contenant par exemple, moins de 2 % de composés C₈ aromatiques et l'on soutire en fond de cette colonne par une ligne 15 un liquide (raffinat débarrassé du solvant) riche en éthylbenzène, en métaxylène et en orthoxylène et appauvri en paraxylène (moins de 3 % par exemple) que l'on envoie dans une unité d'isomérisation 21. Ce raffinat est mis en contact avec de l'hydrogène introduit par une ligne 20 et avec un catalyseur à base de mordénite et de platine sur alumine à environ 380°C. Une ligne 22 conduit l'isomérat de la sortie du réacteur vers un ballon de séparation des constituants gazeux (non représenté sur la figure), puis vers une colonne à distiller 23 (température de tête 90°C, température de fond 160°C par exemple). On soutire en tête par une ligne 24 des hydrocarbures de C₁ à C₅, de l'hexane, du cyclohexane, du benzène et du toluène et en fond de cette colonne par une ligne 2, un effluent contenant 8 à 13% d'éthylbenzène, 21 à 24% de paraxylène, 21 à 24% d'orthoxylène, de 45 à 50% de métaxylène et des impuretés, qui est recyclé vers la zone d'adsorption 8.
La ligne 9 introduit l'extrait dans une colonne à distiller 16 d'où l'on soutire en tête du toluène à moins de 2 % de composés C₈ aromatiques (environ 70 % de la quantité introduite dans l'adsorption par exemple) qui est recyclé par des lignes 17 et 11 vers l'alimentation en solvant de désorption de l'unité d'adsorption. En fond de colonne 16 à environ 160°C on soutire un courant enrichi en paraxylène (à environ 90% de paraxylène) au moyen d'une ligne 19 qui le conduit dans une unité de cristallisation 5a à un étage par exemple fonctionnant à environ -10°C. Dans cette unité 5a, 5b on produit des cristaux de paraxylène qui sont en suspension dans une liqueur mère. Les cristaux sont séparés dans au moins une centrifugeuse 5b par exemple et lavés dans la centrifugeuse. On recueille d'une part une liqueur mère appauvrie en paraxylène (environ 54 %) qui est recyclée par la ligne 3 vers la zone d'adsorption 8 via des zones de traitement à la terre et de distillation discutées plus loin et d'autre part des cristaux de paraxylène, que l'on fond. Le solvant de lavage, le toluène par exemple est amené par une ligne 18 et peut provenir comme représenté sur la figure, de l'unité de distillation du raffinat 12 et/ou encore de l'unité de distillation de l'extrait 16. On récupère de l'unité 5b, et après une distillation des cristaux fondus non représentée, du paraxylène liquide de pureté par exemple égale à 99,75% par une ligne 25 et du toluène que l'on recycle (ligne non représentée).

Selon une variante non illustrée, la centrifugeuse peut être remplacée par au moins une colonne de séparation et de lavage à contre-courant, décrite dans les brevets US 4 475 355, US 4 481 169, la colonne NIRO par exemple. Dans ce cas, la liqueur mère et la liqueur de lavage sont une seule et même liqueur qui est éventuellement distillée avant d'être recyclée, en partie au moins, vers la zone d'adsorption, via les zones de traitement à la terre et de distillation, et en partie éventuellement dans la zone de cristallisation.

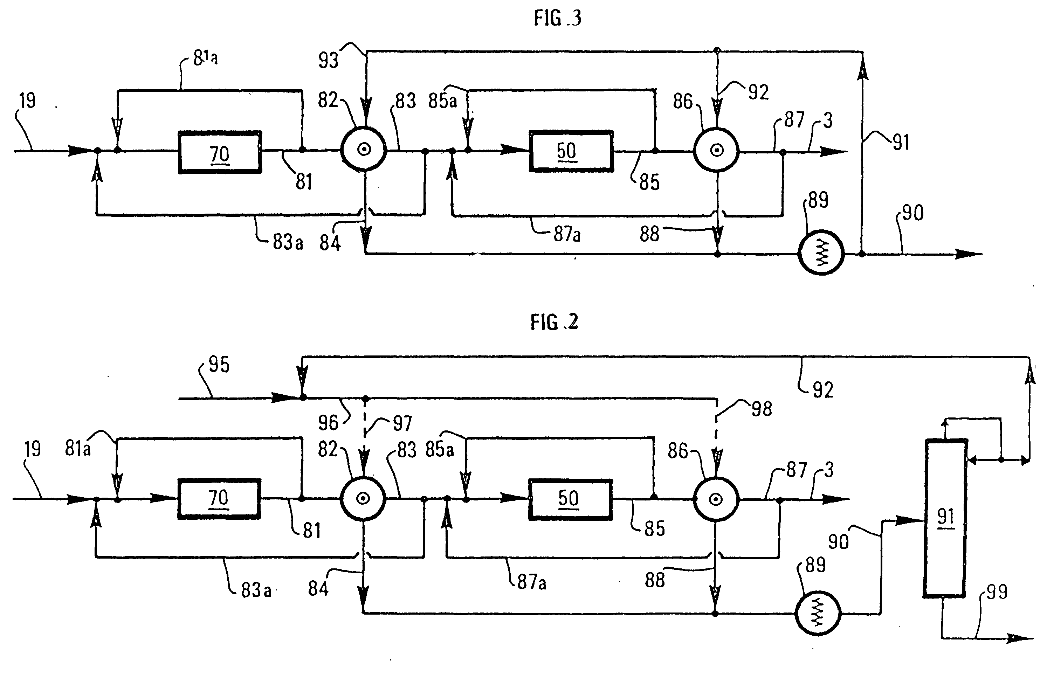

Les figures 2 et 3 illustrent une cristallisation du paraxylène à deux étages, la température du second étage de cristallisation de la liqueur mère étant plus basse que celle du premier étage.

Selon la figure 2, la charge de cristallisation (l'extrait distillé de l'adsorption de pureté égale à 80%) est introduite par la ligne 19 dans une première unité de cristallisation 70 qui fonctionne vers 0°C. On récupère par une ligne 81 des premiers cristaux en suspension dans une première liqueur mère, qui sont séparés dans au moins une première centrifugeuse 82, lavés par du toluène amené par une ligne 97 et récupérés par une ligne 84. La première liqueur mère contenant par exemple 70 % de paraxylène est introduite au moins en partie, par une ligne 83 dans une seconde unité de cristallisation 50 opérant à -10° C. Une autre partie, peut être recyclée dans la première unité de cristallisation par une ligne 83a. On récupère par une ligne 85 des seconds cristaux en suspension dans une seconde liqueur mère, et on les sépare dans au moins une seconde centrifugeuse 86. On les recueille par une ligne 88 après les avoir lavés par du toluène que' l'on a introduit par une ligne 98 dans la seconde centrifugeuse . La seconde liqueur mère recueillie par la ligne 87 contient une fraction du toluène de lavage ; elle est recyclée au moins en partie vers la zone d'adsorption 8 via les zones de traitement à la terre et de distillation et éventuellement en partie par une ligne 87a vers la seconde unité de cristallisation.

Les premiers et seconds cristaux de paraxylène sont mélangés et introduits dans une zone de fusion 89. Une ligne 90 collecte un courant de paraxylène fondu que l'on introduit dans une colonne de distillation 91 qui délivre, en fond, le paraxylène de très haute pureté et, en tête, du toluène que l'on recycle par une ligne 92 et que l'on mélange à un appoint de toluène apporté par une ligne 95 ou une ligne 18. Le mélange de toluène obtenu est introduit au moins en partie dans chacune des centrifugeuses 82 et 86, comme solvant de lavage.

La figure 3 reprend les mêmes organes et les mêmes références que la figure 2, sauf que pour l'étape de lavage des cristaux, on utilise comme solvant de lavage, du paraxylène fondu très pur récupéré à partir de la ligne 90. En effet, une partie au moins du paraxylène fondu très pur est prélevé par une ligne 91 et introduit dans la première centrifugeuse 82 et dans la seconde centrifugeuse 86 pour laver respectivement les premiers et seconds cristaux. La première liqueur mère et une première liqueur de lavage sont envoyées par la ligne 83 dans la seconde unité de cristallisation 50 tandis que la seconde liqueur mère et la seconde liqueur de lavage sont collectées par la ligne 87 pour être recyclées au moins en partie vers la zone d'adsorption 8.

On a décrit dans cette figure l'utilisation de centrifugeuses 82 et 86 pour séparer les cristaux des liqueurs mère et les laver mais on pourrait aussi bien les remplacer par des colonnes à contre courant, type colonne NIRO. Dans ce cas, les solutions respectives recueillies rassembleraient la liqueur mère et la liqueur de lavage provenant de chaque colonne.

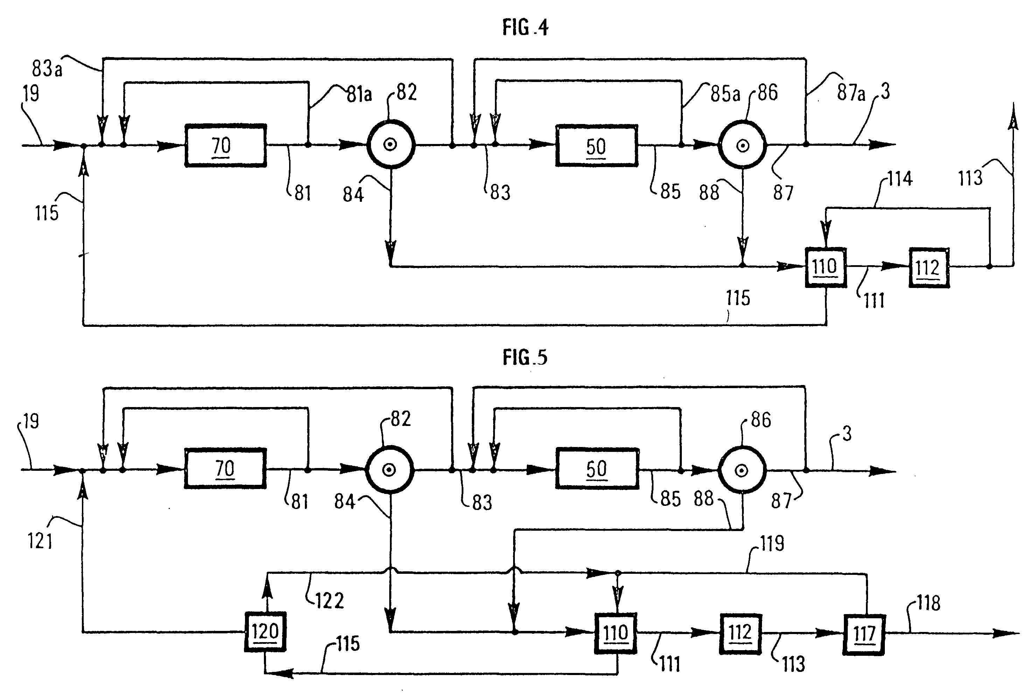

La figure 4 représente une variante préférée du procédé à deux étages de cristallisation opérant avantageusement entre +5 et - 7° C pour le premier et entre -7 et -25° C pour le second.
La charge de cristallisation (l'extrait distillé de l'adsorption) est introduite par la ligne 19 dans une première unité de cristallisation 70. On récupère par une ligne 81 des premières cristaux en suspension dans une première liqueur mère qui sont séparés dans au moins une centrifugeuse 82 ou au moins un filtre rotatif. La première liqueur mère recueillie par la ligne 83 est introduite au moins en partie dans une seconde unité 50 de cristallisation, une autre partie pouvant être recyclée vers la première unité de cristallisation 70. On recueille une seconde suspension de cristaux par une ligne 85 que l'on sépare dans au moins une centrifugeuse ou filtre rotatif 86. Une seconde liqueur mère est soutirée par une ligne 87 et recyclée au moins en partie vers la zone d'adsorption 8 via les zones de traitement à la terre et de distillation, une autre partie pouvant être prélevée et recyclée vers la seconde unité de cristallisation par une ligne 87a raccordée à la ligne 87. Les premiers et seconds cristaux respectivement recueillis par des lignes 84 et 88 sont rassemblés et introduits dans au moins une colonne de lavage 110 type colonne NIRO où ils sont lavés par un solvant de lavage. On recueille des cristaux de paraxylène par une ligne 111 qui sont fondus complètement dans une zone de fusion 112 et on soutire un courant de paraxylène de très haute pureté. Une partie du courant de paraxylène est prélevé par une ligne 114 et introduite comme solvant de lavage dans la colonne 110. La liqueur de lavage recueillie dan la colonne est recyclée au moins en partie vers la première unité de cristallisation.

Selon la figure 5, lorsque le solvant de lavage dans la colonne de lavage est le solvant de désorption (toluène) ou un autre solvant approprié comme le pentane, le courant de paraxylène fondu contenant une mineure partie de solvant peut être distillé dans une unité de distillation 117. Le paraxylène de très haute pureté est recueilli par une ligne 118 tandis que la fraction légère comprenant le solvant de lavage est recyclée dans la colonne NIRO. Enfin, la liqueur de lavage soutirée par la ligne 115 et contenant du solvant est distillée dans une unité de distillation 120, le solvant est recyclé au moins en partie dans la colonne et la liqueur de lavage débarrassée de la majeure partie du solvant est recyclée au moins en partie vers la première unité de cristallisation par la ligne 121.

Il a été mentionné pour une figure donnée le recyclage de la liqueur mère provenant d'un étage A de cristallisation dans ledit étage A ou d'un étage B dans ledit étage B. Il est bien entendu que ces recyclages peuvent s'appliquer pour toutes les figures.

De même, il est connu qu'une suspension de cristaux provenant d'un étage de cristallisation peut être recyclée vers cet étage et ce recyclage peut s'appliquer aussi à toutes les figures (81a et 85a).

On a souvent utilisé dans la description le terme d'une zone de séparation. Bien entendu, il s'agit d'au moins une centrifugeuse ou d'au moins un filtre rotatif ou d'au moins une colonne de lavage à contre courant par un solvant.

La zone de séparation où s'effectue le lavage des cristaux peut comprendre au moins une centrifugeuse ou au moins un filtre rotatif. On a cependant remarqué qu'en utilisant, comme zone de séparation où s'effectue le lavage des cristaux au moins une colonne de lavage à contre courant, type NIRO par exemple et en particulier, avec une partie du paraxylène fondu très pur recueilli, en tant que solvant de lavage, on obtenait d'excellents résultats et on diminuait le coût des utilités.

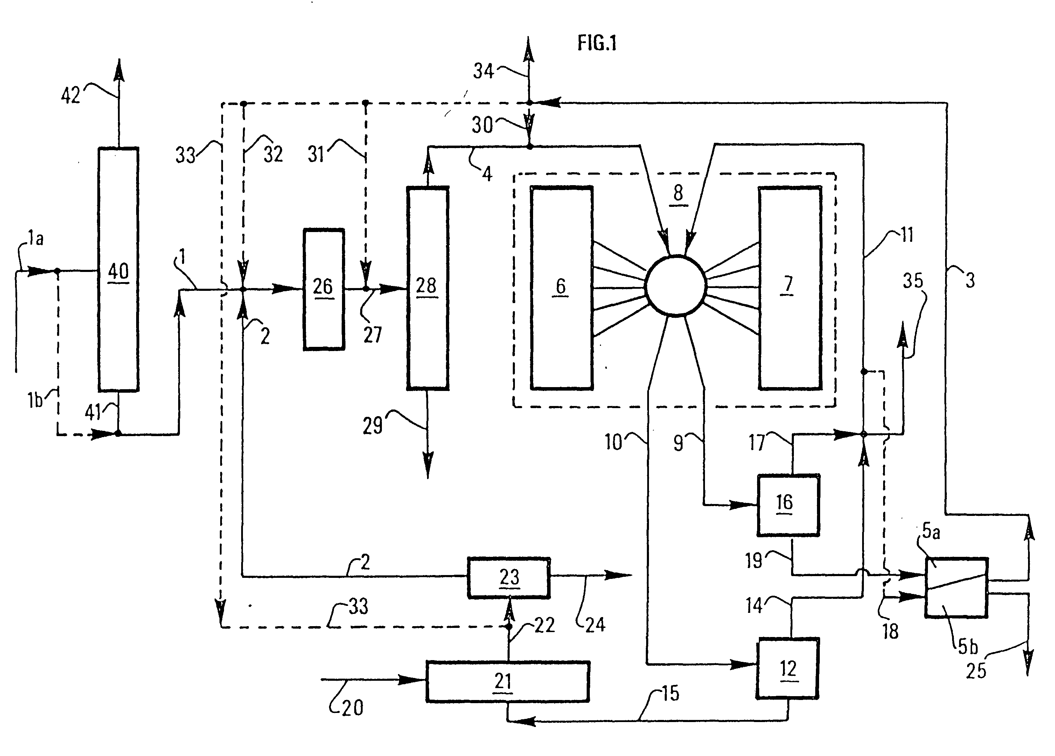

Comme on l'a schématisé sur la figure 1, la liqueur mère provenant de l'unité de cristallisation 5b est recyclée vers l'unité d'adsorption 8. Dans le cas d'une cristallisation à deux étages ou à plusieurs étages, la liqueur mère provient de l'étage le plus froid de cristallisation après la séparation des cristaux de paraxylène (ligne 87, figures 7 à 9). Les impuretés qui circulent dans la boucle du dispositif adsorption, cristallisation, isomérisation peuvent être des hydrocarbures oléfiniques, ainsi que des hydrocarbures paraffiniques et naphténiques ou autres composés oxygénés. Elles peuvent provenir notamment, aussi bien de la charge à traiter qui provient d'un réformage catalytique que de l'isomérisation. Ces impuretés circulent donc et peuvent se retrouver dans toutes les fractions et notamment dans l'extrait et donc dans la liqueur mère résultant de l'étape de cristallisation. Cette liqueur mère peut être introduite par une ligne 32 reliée à la ligne 3 et à la ligne 53 ou 87 dans au moins un réacteur 26 de traitement à la terre, avantageusement deux, disposé en amont et relié par une ligne 27 à l'unité d'adsorption 8. A cette ligne 32 peuvent être raccordées la ligne 1 contenant la charge à traiter et la ligne 2 contenant l'isomérat, les trois flux étant ainsi traités en mélange dans un réacteur unique 26.

Selon une autre variante, la charge 1 peut avoir été prétraitée dans un autre réacteur de traitement à la terre (non indiqué sur la figure). Il en est de même pour l'isomérat 2 qui peut aussi avoir été initialement prétraité après son passage dans l'unité de distillation 23.
Selon une variante préférée, la liqueur mère 3 peut être introduite directement dans la ligne 22 conduisant à l'unité de distillation 23 de l'isomérat, avant d'être traitée, en mélange avec l'isomérat distillé, dans le réacteur 26 de traitement à la terre. Cette variante permet d'éliminer sensiblement tous les composés les plus volatils non seulement de l'isomérat mais de la liqueur mère.

Lorsque l'unité de distillation est réglée pour délivrer aussi, en fond, une fraction supplémentaire contenant la majorité des composés lourds (hydrocarbures en C9+, aldéhydes, cétones), le traitement à la terre du mélange distillé comprenant l'isomérat et la liqueur mère s'en trouve substantiellement amélioré.

Une partie de la liqueur mère peut aussi être recyclée par une ligne 31 à l'effluent 27 du réacteur 26.

L'effluent du réacteur de traitement à la terre et éventuellement la liqueur mère de cristallisation 31 pouvant contenir encore des hydrocarbures lourds tels que des hydrocarbures à neuf atomes de carbone sont introduits par une ligne 27 dans une colonne à distiller 28 qui délivre, en fond de colonne (ligne 29), les impuretés indésirables et en tête un distillat correspondant à la coupe C₈ purifiée, que l'on introduit par la ligne 4 à l'unité d'adsorption 8. Une partie de la liqueur mère peut aussi être introduite dans la ligne 4 par une ligne 30.

Ces divers recyclages peuvent être combinés entre eux, lorsqu'il s'agit, par exemple, de réutiliser des équipements existants pour la distillation 23, le traitement à la terre 26 ou la distillation 28 et lorsque l'un de ces équipements est déjà opéré à son débit maximum ou bien encore lorsque l'on cherche à faire baisser la teneur en une impureté dans la boucle sans chercher à l'éliminer totalement. En d'autres termes, la liqueur mère de l'unité de cristallisation (étage le plus froid), véhiculée par la ligne 3 peut être en partie recyclée vers l'unité d'adsorption 8 soit directement au moyen de la ligne 30, soit indirectement au moyen des lignes 31, 32 ou 33.

De manière à contenir la teneur en constituants de volatilité intermédiaire du toluène (solvant de desorption) à un niveau tolérable, par exemple inférieur à 5 %, on effectue au moins une purge de toluène pollué par lesdits constituants au moyen d'une ligne 35 reliée soit à la ligne 17, soit à la ligne 14 soit à la ligne 11 collectant l'ensemble du solvant recyclé vers l'unité d'adsorption 8.
De plus, on peut effectuer une purge de la liqueur mère issue de la cristallisation si la teneur en constituants de volatilité intermédiaire y est trop forte. Cette purge est réalisée au moyen de la ligne 34 raccordée à la ligne 3.

La purge de toluène peut être compensée par un appoint de toluène. Étant donné que les sources les plus importantes de coupe C₈ aromatique (ligne la) proviennent du reformage catalytique, de la dismutation (disproportionnation) du toluène en benzène et xylène et de la transalkylation toluène - C₉ aromatique et que les effluents de ces unités dont il proviennent sont en partie généralement purifiés dans un train d'unités de distillation dont la colonne 28 peut faire partie, on peut utiliser au moins en partie comme source d'appoint de toluène, soit celui produit en tête (ligne 42) d'une colonne de distillation 40 de toluène en avant du réacteur 26 contenant la terre soit celui qui résulte du by-pass par une ligne 1b d'une partie au moins de la charge (ligne 1) que l'on mélange à l'effluent de fond de la colonne 40 soit celui qui est introduit dans la charge purifiée (ligne 1) par le déréglage de la colonne 40 qui permet de laisser passer dans la coupe C8 la proportion voulue de toluène.

## Revendications

1. Procédé de production de paraxylène de très haute pureté à partir d'une charge contenant un mélange d'hydrocarbures aromatiques de 7 à 9 atomes de carbone dans lequel on fait circuler une partie au moins de la charge dans une zone dite d'enrichissement adaptée à enrichir une première fraction à plus de 50 % en poids de paraxylène et on purifie au moins une partie de ladite première fraction dans une zone dite de purification par au moins deux cristallisations à haute température comprise entre +10°C et -30°C dans au moins deux zones de cristallisation, le procédé étant **caractérisé en ce que** l'on cristallise ladite première fraction enrichie en paraxylène dans une première zone de cristallisation (70) à une température T1, on sépare les premiers cristaux d'une première liqueur mère, on récupère des premiers cristaux de paraxylène, on cristallise une partie au moins de la première liqueur mère dans une seconde zone de cristallisation (50), à une température T2 inférieure à la température T1, on sépare des seconds cristaux d'une seconde liqueur mère, on récupère des seconds cristaux de paraxylène et on mélange les premiers et les seconds cristaux de paraxylène.

2. Procédé selon la revendication 1 dans lequel la zone d'enrichissement en paraxylène est au moins une zone de cristallisation à très basse température dans laquelle est introduite la charge contenant un mélange d'hydrocarbures aromatiques à 8 atomes de carbone qui délivre, après une étape de séparation, un liquide que l'on isomérise dans une zone d'isomérisation et des cristaux que l'on fond qui sont une partie au moins de ladite première fraction.

3. Procédé selon la revendication 1 dans lequel la zone d'enrichissement en paraxylène est une zone (8) d'adsorption sélective contenant un adsorbant zéolithique dans laquelle est introduite la charge contenant un mélange d'hydrocarbures aromatiques à 8 atomes de carbone, on réalise une adsorption sélective de la charge, en présence d'un solvant de désorption, on récupère ladite première fraction (9, 19) enrichie en paraxylène et une seconde fraction (10, 15) appauvrie en paraxylène, on isomérise ladite seconde fraction dans une zone (21) d'isomérisation contenant un catalyseur d'isomérisation dans des conditions adéquates pour produire un isomérat contenant du paraxylène et on recycle (2) au moins en partie l'isomérat vers la zone d'adsorption.

4. Procédé selon l'une des revendications 1 à 3 dans lequel une partie au moins de la liqueur mère résultant de l'étage le plus froid de la zone de purification est recyclée vers la zone (8) d'enrichissement.

5. Procédé selon la revendication 1 dans lequel la zone d'enrichissement en paraxylène est au moins une zone de dismutation de la charge consistant essentiellement en toluène produisant un effluent contenant du benzène et des xylènes et qu'on distille pour le débarrasser du benzène et du toluène n'ayant pas réagi.

6. Procédé selon l'une des revendications 1 à 5, dans lequel on effectue (figures 2 et 3) une première cristallisation de la fraction enrichie en paraxylène dans une première zone (70) de cristallisation à une température T1, on récupère une suspension (81) de premiers cristaux de paraxylène dans une première liqueur mère, on sépare les premiers cristaux de la première liqueur mère dans une première zone (82) de séparation, on lave les premiers cristaux avec un solvant de lavage dans la première zone de séparation, on cristallise une partie au moins de la première liqueur mère et d'une liqueur de lavage dans la seconde zone (50) de cristallisation à une température T2 inférieure à la température T1, on récupère une suspension (85) de seconds cristaux de paraxylène dans une seconde liqueur mère, on sépare les seconds cristaux de la seconde liqueur mère dans une seconde zone (86) de séparation, on lave les seconds cristaux avec le solvant de lavage dans la seconde zone de séparation, on récupère une seconde liqueur mère (87) contenant de la liqueur de lavage , on mélange les premiers et les seconds cristaux de paraxylène, on les fond (89) complètement et on récupère un courant (90) de paraxylène fondu.

7. Procédé selon l'une des revendications 1 à 5, dans lequel on effectue (figures 4 et 5) une première cristallisation de la fraction enrichie en paraxylène dans une première zone de cristallisation à une température T1, on récupère une suspension de premiers cristaux de paraxylène dans une première liqueur mère, on sépare les premiers cristaux de la première liqueur mère dans une première zone (82) de séparation, on cristallise une partie au moins de la première liqueur mère dans une seconde zone (50) de cristallisation à une température T2 inférieure à T1, on sépare les seconds cristaux de la seconde liqueur mère dans une seconde zone (86) de séparation, on récupère la seconde liqueur mère, on récupère les premiers cristaux et les seconds cristaux et on les lave dans au moins une zone (110) de séparation et de lavage avec un solvant de lavage approprié, on récupère d'une part une liqueur (115) de lavage qui est recyclée au moins en partie vers la première zone (70) de cristallisation, on récupère d'autre part des cristaux de paraxylène, on les fond complètement dans une zone de fusion (112) et on recueille un courant de paraxylène fondu.

8. Procédé selon l'une des revendications 6 et 7, dans lequel le solvant de lavage est du paraxylène fondu.

9. Procédé selon la revendication 6 (fig. 2), dans lequel le solvant de lavage est un solvant autre que le paraxylène fondu, on récupère (90) un courant de paraxylène fondu contenant du solvant, on distille (91) ledit courant, on récupère du paraxylène pur (99) et du solvant (92) que l'on recycle dans chacune des zones de séparation (82,86).

10. Procédé selon la revendication 7 (fig. 5), dans lequel le solvant de lavage est un solvant autre que du paraxylène fondu, on récupère la liqueur de lavage (115) qui est distillée, le solvant (122) récupéré est recyclé dans la zone de séparation et de lavage et la liqueur de lavage (121) débarrassée du solvant est recyclée vers la première zone de cristallisation.

11. Procédé selon l'une des revendications 7 et 10, dans lequel la zone de séparation et de lavage est une colonne à contre-courant.

## Patentansprüche

1. Verfahren zur Erzeugung von Paraxylol sehr hoher Reinheit ausgehend von einer ein Gemisch von aromatischen Kohlenwasserstoffen mit 7 bis 9 Kohlenstoffatomen enthaltenden Charge, bei dem man wenigstens einen Teil der Charge in einer sog. Anreicherungszone zirkulieren lässt, die so ausgelegt ist, dass sie eine erste Fraktion von mehr als 50 Gew.-% Paraxylol anreichert und wenigstens einen Teil dieser ersten Fraktion in einer sog. Reinigungszone durch wenigstens zwei Kristallisationen bei hoher Temperatur zwischen +10°C und -30°C in wenigstens zwei Kristallisationszonen reinigt, **dadurch gekennzeichnet, dass** man diese erste an Paraxylol angereicherte Fraktion in einer ersten Kristallisationszone (70) bei einer Temperatur T1 kristallisiert, die ersten Kristalle von einer ersten Stammlösung trennt, erste Paraxylolkristalle gewinnt, wenigstens einen Teil der ersten Stammlösung in einer zweiten Kristallisationszone (50) bei einer Temperatur T2 unterhalb der Temperatur T1 kristallisiert, zweite Kristalle einer zweiten Stammlösung abtrennt und zweite Paraxylolkristalle gewinnt und die ersten und zweiten Paraxylolkristalle vermischt.

2. Verfahren nach Anspruch 1, bei dem die Anreicherungszone an Paraxylol wenigstens eine Kristallisationszone von sehr niedriger Temperatur ist, in die die ein Gemisch aus aromatischen Kohlenwasserstoffen mit 8 Kohlenstoffatomen enthaltende Charge eingeführt wird, welches nach einer Trennstufe eine Flüssigkeit, die man in einer Isomerierungszone isomeriert und Kristalle, die man schmilzt und die ein Teil wenigstens dieser ersten Fraktion sind, liefert.

3. Verfahren nach Anspruch 1, bei dem die Anreichungszone an Paraxylol eine Zone (8) der selektiven ein zeolithisches Adsorbens enthaltende Adsorption (8) ist, in welche die ein Gemisch aus aromatischen Kohlenwasserstoffen mit acht Kohlenstoffatomen enthaltende Charge eingeführt wird, man eine selektive Adsorption der Charge in Gegenwart eines Desorptionslösungsmittels realisiert, die die erste an Paraxylol angereicherte Fraktion (9, 19) und eine zweite an Paraxylol verarmte Fraktion (10, 15) gewinnt, man diese zweite Fraktion in einer Isomerierungszone (21) isomeriert, die einen Isomerierungskatalysator unter adäquaten Bedingungen enthält, um ein Paraxylol enthaltendes Isomerat zu erzeugen und man rezykliert (2) wenigstens zum Teil das Isomerat zur Adsorptionszone.

4. Verfahren nach einem der Ansprüche 1 bis 3, bei dem wenigstens ein Teil der Stammlösung, die aus der kältesten Stufe der Reinigungszone stammt, zur Anreichrungszone (8) rezykliert wird.

5. Verfahren nach Anspruch 1, bei dem die Zone der Anreicherung an Paraxylol wenigstens eine Dismutationszone für die Charge ist, die im wesentlichen aus Toluol besteht und einen Bezol- und Xylole enthaltenden Abstrom erzeugt und den man destilliert, um ihn vom Benzol- und Toluol, die nicht reagiert haben, zu befreien.

6. Verfahren nach einem der Ansprüche 1 bis 5, bei dem man (Figuren 2 und 3) eine erste Kristallisation der an Paraxylol angereicherten Fraktion in einer ersten Kristallisationszone (70) bei einer Temperatur T1 vornimmt, eine Suspension (81) aus ersten Paraxylolkristallen in einer Stammlösung gewinnt, die ersten Kristalle der Stammlösung in einer ersten Trennzone (82) trennt, die ersten Kristalle mit einer Waschlösung in der ersten Trennzone wäscht, wenigstens einen Teil der ersten Stammlösung und einer Waschflüssigkeit in der zweiten Kristallisationszone (50) bei einer Temperatur T2 unterhalb der Temperatur T1 kristallisiert, eine Suspension (85) aus zweiten Paraxylolkristallen in einer zweiten Stammlösung gewinnt, die zweiten Kristalle der zweiten Stammlösung in einer zweiten Trennzone (86) abtrennt, die zweiten Kristalle mit dem Waschlösungsmittel in der zweiten Trennzone wäscht, eine zweite Waschflüssigkeit enthaltende Stammlösung (87) gewinnt und die ersten und zweiten Paraxylolkristalle mischt, man sie vollständig schmilzt (89) und einen Strom (90) schmelzflüssigen Paraxylols gewinnt.

7. Verfahren nach einem der Ansprüche 1 bis 5, bei dem man (Figuren 4 und 5) eine erste Kristallisation der an Paraxylol angereicherten Fraktion in einer ersten Kristallisationszone bei einer Temperatur T1 vornimmt, eine Suspension erster Paraxylolkristalle in einer ersten Stammlösung gewinnt, die ersten Kristalle von der ersten Stammlösung in einer ersten Trennzone (82) abtrennt und wenigstens einen Teil der ersten Stammlösung in einer zweiten Kristallisationszone (50) bei einer Temperatur T2 unterhalb T1 kristallisiert und die zweiten Kristalle der zweiten Stammlösung in einer zweiten Trennzone (86) abtrennt, die zweite Stammlösung gewinnt, die ersten Kristalle und die zweiten Kristalle gewinnt und man sie in wenigstens einer Trenn- und Waschzone (110) mit einem geeigneten Lösungsmittel wäscht, einerseits eine Waschlösung (115) gewinnt, die wenigstens zum Teil zur ersten Kristallisationszone (70) rezykliert wird und andererseits Paraxylolkristalle gewinnt, man sie vollständig in der Schmelzzone (112) schmilzt und einen Strom schmelzflüssigen Paraxylols sammelt.

8. Verfahren nach einem der Ansprüche 6 und 7, bei dem das Waschlösungsmittel schmelzflüssiges Paraxylol ist.

9. Verfahren nach Anspruch 6 (Figur 2), bei dem das Waschlösungsmittel ein Lösungsmittel außer schmelzflüssigem Paraxylol ist, man einen Lösungsmittel enthaltenden Strom schmelzflüssigen Paraxylols gewinnt, man diesen Strom destilliert (91), reines Paraxylol (99) und Lösungsmittel (92) gewinnt, die man in jede der Trennzonen (82, 86) rezykliert.

10. Verfahren nach Anspruch 7 (Figur 5), bei dem das Waschlösungsmittel ein Lösungsmittel außer dem schmelzflüssigen Paraxylol ist, man die Waschflüssigkeit (115), die destilliert wird, gewinnt, wobei das gewonnene Lösungsmittel (122) in die Trenn- und Waschzone rezykliert wird und die vom Lösungsmittel befreie Waschlösung (121) zur ersten Kristallisationszone rezykliert wird.

11. Verfahren nach einem der Ansprüche 7 und 10, bei dem die Trenn- und Waschzone eine Gegenstromkolonne ist.

## Claims

1. Process for the production of paraxylene of very high purity from a feedstock that contains a mixture of aromatic hydrocarbons having 7 to 9 carbon atoms in which at least a portion of the feedstock is circulated in a so-called enrichment zone that is suited to enrich a first fraction at more than 50 % by weight of paraxylene, and at least a portion of said first fraction is purified in a so-called purification zone by at least two high-temperature crystallization comprised between + 10°C and - 30°C in at least two crystallization zones, whereby the process is **characterized in that** said first fraction that is enriched with paraxylene is crystallized in a first crystallization zone (70) at a temperature T1, the first crystals are separated from a first mother liquor, first paraxylene crystals are recovered, at least one part of the first mother liquor is crystallized in a second crystallization zone (50) at a temperature T2 lower than the temperature T1, second crystals are separated from a second mother liquor, second paraxylene crystals are recovered and the first and second paraxylene crystals are mixed.

2. Process according to claim 1, wherein the paraxylene-enrichment zone is at least a crystallization zone at a very low temperature in which the feedstock containing the aromatic hydrocarbon mixture with 8 carbon atoms is introduced, which delivers, after a separation stage, a liquid that is isomerized in an isomerization zone and crystals that are melted and that are at least a portion of said first fraction.

3. Process according to claim 1, wherein the paraxylene-enrichment zone is a selective adsorption zone (8) that contains a zeolitic adsorbent in which the feedstock containing the aromatic hydrocarbon mixture with 8 carbon atoms is introduced ; a selective adsorption of the feedstock is carried out; in the presence of a desorption solvent, said first paraxylene-enriched fraction (9, 19) and a second paraxylene-depleted fraction (10, 15) are recovered; said second fraction is isomerized in an isomerization zone (21) that contains a catalyst for isomerization under conditions that are suitable for producing an isomerate that contains paraxylene, and the isomerate is recycled (2) at least in part to the adsorption zone.

4. Process according to one of claims 1 to 3, wherein at least a portion of the mother liquor that results from the coldest stage of the purification zone is recycled to enrichment zone (8).

5. Process according to claim 1, wherein the paraxylene enrichment zone is at least one disproportionation zone of the feedstock consisting essentially in toluene that produces an effluent containing benzene and xylenes and the effluent is distilled for removing benzene and unreacted toluene.

6. Process according to one of claims 1 to 5, wherein a first crystallization of the paraxylene-enriched fraction is carried out (Figures 2 and 3) in a first crystallization zone (70) at a temperature T1; a suspension (81) of first paraxylene crystals is recovered in a first mother liquor; the first crystals from the first mother liquor are separated in a first separation zone (82); the first crystals are washed with a washing solvent in the first separation zone; at least a portion of the first mother liquor and of the washing liquor is crystallized in a second crystallization zone (50) at a temperature T2 that is lower than temperature T1; a suspension (85) of second paraxylene crystals is recovered in a second mother liquor; the second crystals from the second mother liquor are separated in a second separation zone (86); the second crystals are washed with the washing solvent in the second separation zone; a second mother liquor (87) containing washing liquor is recovered ; the first and the second paraxylene crystals are mixed, and they are melted (89) completely, and a stream (90) of molten paraxylene is recovered.

7. Process according to one of claims 1 to 5 wherein a first crystallization of the paraxylene-enriched fraction is carried out (Figures 4 and 5) in a first crystallization zone at a temperature T1; a suspension of first paraxylene crystals is recovered in a first mother liquor ; the first crystals from the first mother liquor are separated in a first separation zone (82); at least a portion of the first mother liquor is crystallized in a second crystallization zone (50) at a temperature T2 that is less than T1, the second crystals from the second mother liquor are separated in a second separation zone (86); the second mother liquor is recovered; the first crystals and the second crystals are recovered and they are washed in at least one zone (110) for separation and washing with a suitable washing solvent; on the one hand, a washing liquor (115) is recovered that is recycled at least in part to first crystallization zone (70), and on the other hand, paraxylene crystals are recovered; they are melted completely in a melting zone (112), and a stream of molten paraxylene is collected.

8. Process according to one of claims 6 and 7, wherein the washing solvent is molten paraxylene.

9. Process according to claim 6 (fig 2), wherein the washing solvent is a solvent other than the molten paraxylene, a stream of molten paraxylene containing solvent is recovered (90) said stream is distilled (91), pure paraxylene (99) and solvent (92) are recovered and the solvent is recycled to each of the separation zones (82, 86).

10. Process according to claim 7 (fig 5), wherein the washing solvent is a solvent other than molten paraxylene, washing liquor (115) is recovered that is distilled, the recovered solvent (122) is recycled to the separation and washing zone and the washing liquor (121) in which the solvent is removed is recycled to the first crystallization zone.

11. Process according to one of claims 7 and 10, wherein the separation and washing zone is a countercurrent column.
